(19) Europäisches Patentamt
European Patent Office
Office européen des brevets

(11) Veröffentlichungsnummer: **0 090 216**
**B1**

(12) # EUROPÄISCHE PATENTSCHRIFT

(45) Veröffentlichungstag der Patentschrift: **13.11.85**

(51) Int. Cl.⁴: **C 07 C 109/10**, C 08 K 5/25, B 01 J 10/00, H 01 B 3/18, H 01 B 3/30

(21) Anmeldenummer: **83102319.7**

(22) Anmeldetag: **09.03.83**

(54) **Verfahren und Vorrichtung zur Herstellung von N,N'-bis-aroyl-hydrazinen.**

(30) Priorität: **24.03.82 DE 3210859**

(43) Veröffentlichungstag der Anmeldung: **05.10.83 Patentblatt 83/40**

(45) Bekanntmachung des Hinweises auf die Patenterteilung: **13.11.85 Patentblatt 85/46**

(84) Benannte Vertragsstaaten: **AT BE CH DE FR GB IT LI LU NL SE**

(56) Entgegenhaltungen:
**EP-A-0 025 505**
**EP-A-0 047 423**
**EP-A-0 071 849**

(73) Patentinhaber: **Siemens Aktiengesellschaft, Berlin und München Wittelsbacherplatz 2, D-8000 München 2 (DE)**

(72) Erfinder: **von Gentzkow, Wolfgang, Dr., Zwetschgenweg 1, D-8524 Kleinsendelbach (DE)**
Erfinder: **Schmiedel, Manfred, Volkacherstrasse 26, D-8500 Nürnberg-Grossbründlach (DE)**
Erfinder: **Tussing, Reinhold, Buchenstrasse 8, D-8627 Redwitz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein Verfahren zur Herstellung von N.N'-Bis-aroyl-hydrazinen durch katalytische Umsetzung von Arylcarbonsäurealkylestern mit Hydrazin oder Aroylhydrazin in der Hitze.

Es ist bekannt, für die Stabilisierung von Polyolefinen gegen den thermooxidativen kupferkatalysierten Abbau Derivate des N.N'-Bis-salicyloyl-hydrazins zu verwenden (US-PS 3 110 696, DE-OS 2 150 131 und 2 703 558).

Nach bekannten Verfahren (DE-OS 2 150 131) werden N.N'-Bis-salicyloyl-hydrazine durch Umsetzung von Salicylsäurehydrazid mit Salicylsäure unter Zugabe von Thionylchlorid und Pyridin in Chlorbenzol erhalten. Die dabei anfallenden Produkte enthalten noch Ausgangskomponenten und müssen durch Umkristallisieren gereinigt werden. So erhaltenes N.N'-Bis-salicyloyl-hydrazin, das als Metalldesaktivator für Polymere eingesetzt wird, kann bei der Herstellung und Verarbeitung Augenreizungen und Augenschädigungen verursachen.

Gemäß dem Verfahren der DE-OS 2 933 870 können N.N'-Bis-salicyloyl-hydrazine hergestellt werden durch Umsetzung von Salicylsäurealkylestern mit Hydrazin bzw. Salicylsäurehydrazid gegebenenfalls in Gegenwart nukleophiler und/oder elektrophiler Katalysatoren. Bei einem so erhaltenen N.N'-Bis-salicyloyl-hydrazin treten die Augenreizungen nurmehr in verminderter Form auf.

Der europäischen Patentanmeldung 0 047 423 kann entnommen werden, daß die Ausbeute an N.N'-Bis-salicyloyl-hydrazin beachtlich erhöht werden kann, wenn Hydrazin bzw. Salicylsäurehydrazid in einem 1- bis 10fachen Überschuß an Salicylsäurealkylestern in Gegenwart eines Halogenids, Hydroxids oder Oxids von Bor, Aluminium oder Zink als Katalysator bei Temperaturen bis zu 150° C umgesetzt werden. Vornehmlich bei Verwendung von Boroxid als Katalysator fällt das Produkt in hoher Reinheit an und ist nach Versuchen am Kaninchenauge noch weniger augenreizend und augenschädigend als die bisher erhaltenen Produkte. Das entstehende N.N'-Bis-salicyloyl-hydrazin liegt am Ende der Reaktion als Kristallbrei in überschüssigem Alkylester vor. Es wird abgesaugt, mit Alkohol gewaschen und im Vakuum getrocknet.

Bei all diesen Verfahren fällt pulverförmiges N.N'Bis-salicyloyl-hydrazin an, das bei der Aufarbeitung und technischen Verarbeitung über Staubbildung ins menschliche Auge gelangen und Augenerkrankungen hervorrufen kann.

Aufgabe der Erfindung ist es, bei der Herstellung und technischen Verwendung von N.N'-Bis-salicyloyl-hydrazinen, beispielsweise als Metalldesaktivatoren, eine Kontamination des Personals zu verhindern. Gleichzeitig soll eine Vereinfachung des technischen Herstellungsverfahrens erreicht werden.

Diese Aufgabe wird dadurch gelöst, daß erfindungsgemäß mit Reaktionspartner, nämlich Hydrazin bzw. Aroylhydrazin mit Arylcarbonsäurealkylester in nahezu stöchiometrischen Mengenverhältnissen in einem inerten, bei Reaktionsbedingungen flüssigen bis wachsartigen aliphatischen Kohlenwasserstoff geringer Flüchtigkeit zu einem pastenförmigen Produkt umgesetzt werden und dieses durch Dispergieren homogenisiert und unter Vakuum von den flüchtigen Reaktionskomponenten befreit wird. Dabei wird eine den Metalldesaktivator enthaltende, durch Nebenprodukte gefärbte Paste erhalten, die als solche in Polymere eingearbeitet werden kann. Die Arylgruppen im Aroylhydrazin und Arylcarbonsäureester sind vorzugsweise Phenyl- oder Naphthylreste, deren H-Atome durch OH, Cl, Br, Alkyl mit 1 bis 18 C-Atomen, Alkenyl mit 3 bis 4 C-Atomen und/oder Alkoxy mit 1 bis 18 Kohlenstoffatomen substituiert sind. Die Arylcarbonsäureester beziehen sich auf Alkyl-Reste von $C_1$ bis $C_5$.

Für einige Anwendungsfälle hat sich eine anschließende Reinigung des pastenförmigen Reaktionsproduktes durch Extraktion günstig erwiesen. Geeignete Extraktionsmittel sind aliphatische Alkohole bis $C_5$. Vorzugsweise wird mit Methyl- und/oder Äthylalkohol extrahiert. Dabei wird eine farblose Paste erhalten.

Die Reaktion wird erfindungsgemäß in einem heizbaren Reaktor durchgeführt. Die Ausgangskomponenten werden zusammen mit dem inerten, bei Reaktionsbedingungen flüssigen bis wachsartigen, aliphatischen Kohlenwasserstoff geringer Flüchtigkeit in den Reaktor gefüllt. Der nach Erhitzen bei der ablaufenden Reaktion entstehende Alkohol destilliert kontinuierlich über eine Kolonne ab und erlaubt eine Verfolgung des Reaktionsablaufes. Nach Abschluß der Reaktion wird die erhaltene Kristallpaste unter Vakuum aus dem Reaktor über einen Dispergator in den Kopf des Reaktors zurückgepumpt. Beim Eintritt in den Reaktor wird die Paste über einen am Rührer befestigten Schleuderschirm geführt. Die Paste läuft dann als dünner Film an der Reaktorwand entlang und wird hierbei entgast und von den Resten der flüchtigen Reaktionsprodukte befreit. Danach wird durch Umschalten eines Dreiwegeventils direkt in die Liefergebinde abgefüllt. Bei der mit einer nahezu quantitativen Ausbeute verlaufenden Reaktion können alle Verfahrensschritte bis zum Abfüllen in einer Vorrichtung ohne die Gefahr der Kontamination von Personen durchgeführt werden.

Das erfindungsgemäße Herstellungsverfahren wird dem jeweiligen Verwendungszweck angepaßt; das Endprodukt kann in ungereinigtem Zustand verwendet werden, es kann aber auch bei Bedarf im Reaktor gereinigt werden.

Geeignete, bei Reaktionsbedingungen flüssige bis wachsartige, aliphatische Kohlenwasserstoffe geringer Flüchtigkeit sind z. B. Extenderöle, Petrolate, Paraffine, Mineralöle, synthetische Öle, Wachse und niedermolekulare Olefinpolymere, insbesondere Polyisobuten.

Geeignete Katalysatoren sind nukleophile und insbesondere elektrophile, wie z. B. Halogenide,

Hydroxide und Oxide von Bor, Aluminium und Zink.

Gegenstand der Erfindung ist ferner eine Vorrichtung zur Durchführung des vorstehend gekennzeichneten Verfahrens, die gekennzeichnet ist durch einen Reaktor 1 zur Umsetzung von Hydrazin bzw. Aroylhydrazin und Arylcarbonsäurealkylester in einem inerten, bei Reaktionsbedingungen flüssigen bis wachsartigen Medium geringer Flüchtigkeit und zur Reinigung der Reaktionsprodukte durch Einrichtungen 2 zum Umpumpen, Abtrennen und Abfüllen, durch einen Dispergator 11 zum Homogenisieren der Kristallpaste, durch eine Schleuderscheibe 13 zur Herstellung eines dünnen Films zum Entgasen und Entfernen der flüchtigen Bestandteile und gegebenenfalls durch einen dazwischengeschalteten Separator 15.

In der Zeichnung ist das Schema einer Vorrichtung wiedergegeben, an dem die Erfindung einfach zu demonstrieren ist.

Die erfindungsgemäße Vorrichtung besteht im wesentlichen aus einem Reaktor 1 und einem damit verbundenen Abtrenn-, Abfüll- und Umpumpsystem, das einen Separator 15 aufweisen kann, der mit Dreiwegehähnen 16 und 17 mit dem Umpumpsystem verbunden ist. Der Reaktor 1 ist versehen mit einer vakuumdichten Haube 4, einer vakuumdichten Drehdurchführung 7 mit Rührer 14 und Schleuderscheibe 13, einem Einfüllstutzen 5, einer Öffnung 6 für den Destillieraufsatz und einem Abfüll- bzw. Einlaßrohr 8. Der Reaktor ist beheiz- und kühlbar. Mit 9 ist ein Bodenventil bezeichnet, das zum Umpumpen geöffnet wird. 10 ist eine Schneckenpumpe und 11 ein Dispergator. 12 ist ein Dreiweghahn, der das wahlweise Umpumpen und Abfüllen des pastenförmigen Reaktionsproduktes ermöglicht.

Aufwendiger personeller Arbeitsschutz und Filteranlagen für die Abluft entfallen beim Betrieb der erfindungsgemäßen Vorrichtung. Das erfindungsgemäß hergestellte, als Paste anfallende Produkt kann direkt in Polymere eingearbeitet werden, die gegen katalytische Einflüsse von Kupfer zu schützen sind. Die erfindungsgemäß hergestellten Produkte werden so mit besonderem Vorteil als Metalldesaktivatoren in mit Kupfer oder -legierungen in Berührung stehenden Kohlenwasserstoffverbindungen, wie z. B. Polyolefinen für Kabel- und Leitungsisolierungen, Schmierstoffen und Kühlölen auf Mineralölbasis verwendet. Die Einarbeitung der pastösen, den Metalldesaktivator enthaltenden Masse in Polyolefine kann nach üblichen Methoden durch direktes Einmischen erfolgen, gegebenenfalls auch zusammen mit sonstigen Zusätzen, wie Oxidationsinhibitoren, Weichmachern, Antistatika, Flammschutzmitteln, Pigmenten, Füllstoffen und dergleichen. Es wird hierdurch auf einfache Weise eine sehr schnelle und homogene Verteilung des Metalldesaktivators im Polyolefin erreicht.

Die Erfindung wird anhand der Beispiele noch näher erläutert.

## Beispiel 1

0,5 kg (10 Mol) Hydrazinhydrat werden mit 3,040 kg (20 Mol) Salicylsäuremethylester und 2,714 kg Vaseline unter Schutzgas in der erfindungsgemäßen Vorrichtung erhitzt. Das entstehende Wasser-Methanol-Gemisch wird kontinuierlich aus dem Reaktionsgemisch abdestilliert. Nach ca. drei Stunden ist die Reaktionstemperatur auf 120°C gestiegen und ca. die Hälfte der theoretischen zu erwartenden Menge Methanol und das gesamte Wasser abdestilliert (0,5 kg). Das Hydrazid liegt als Kristallbrei in der Vaseline vor. Nach Zugabe von 30 g Bortrioxid wird weiter erhitzt und die Temperatur auf 150°C gehalten. Das restliche Methanol (0,320 kg) destilliert ab. Nach ca. drei Stunden ist die Reaktion abgeschlossen (erkenntlich an der abdestillierten Methanolmenge).

Die Reaktionsapparatur wird jetzt evakuiert, das Bodenventil geöffnet und der Kristallbrei über den Dispergator umgepumpt. Es erfolgt eine Zerkleinerung der Kristalle. Auf dem Schleuderschirm und an dem oberen Teil der Kesselwandung findet jetzt die Entgasung und die Entfernung des restlichen Methanols aus dünner Schicht statt. Der Dispergator homogenisiert die Paste.

Während des Umpumpens wird die Temperatur auf 80°C erniedrigt. Nach einer Stunde, wenn die Paste homogen und von allen flüchtigen Bestandteilen befreit ist, wird durch Umstellen eines Dreiweghahnes direkt in die Verpackung gefördert.

Ausbeute 80%, bezogen auf N.N'-Bis-salicyloyl-hydrazin.

## Beispiel 2

50 Gew.-Teile Hydrazinhydrat werden mit 304 Gew.-Teilen Salicylsäuremethylester in 230 Gew.-Teilen Polyisobuten (Viskosität bei 20°C = 22 000 mPas) unter kräftigem Rühren auf ca. 90°C erhitzt (Rückflußkühler). Nach 2 Stunden wird Wasser und gebildetes Methanol über eine Kolonne abdestilliert, die Reaktionsmischung mit 3 Gew.-Teilen Bortrioxid versetzt und unter Rühren auf eine Temperatur von ca. 140—150°C erhitzt. Während des weiteren Verlaufes der Umsetzung entstehendes Methanol wird kontinuierlich über eine Kolonne abdestilliert. Nach ca. 3 Stunden ist die Reaktion beendet. Die nun dickflüssige Reaktionsmischung wird auf 50—60°C abgekühlt und bis zur Entfärbung mit Alkohol gewaschen. Die gelbgefärbte Alkoholphase wird von der zähflüssigen Paste abgegossen.

Nach dem Abtrennen des Waschalkohols wird evakuiert und bei ca. 100°C getrocknet. Es werden

460 Gew.-Teile einer farblosen, zähflüssigen Paste erhalten, bestehend aus 230 Gew.-Teilen N.N'-Bis-salicyloyl-hydrazin (85%) und 230 Gew.-Teilen Polyisobuten. (Durch Lösen des Polyisobutens mit Methylenchlorid und Auskochen des zurückbleibenden Feststoffes mit Methanol wird N.N'-Bis-salicyloyl-hydrazin mit einem Schmelz- und Zersetzungsbereich von 305—310°C erhalten.)

Beispiel 3

152 Gew.-Teile Salicylsäurehydrazid werden mit 152 Gew.-Teilen Salicylsäuremethylester und 3 Gew.-Teilen Bortrioxid in 230 Gew.-Teilen Paraffin (Viskosität bei 60°C = 100 mPas) unter kräftigem Rühren auf 140—150°C erhitzt. Während der Umsetzung entstehendes Methanol wird über eine Kolonne abdestilliert. Nach ca. 3 Stunden ist die Reaktion beendet. Die nun dickflüssige Reaktionsmischung wird auf 50—60°C abgekühlt, mit Alkohol (Methanol oder Äthanol) versetzt und die Mischung ca. 10 min bei 50—60°C kräftigst gerührt. Die gelb gefärbte Alkoholphase wird von der zähflüssigen Paste abgegossen und der Vorgang bis zur Entfärbung wiederholt. Nach dem Abtrennen des Waschalkohols wird im Vakuum bei ca. 100°C getrocknet. Es werden 460 Gew.-Teile einer farblosen, zähflüssigen Paste erhalten, bestehend aus 230 Gew.-Teilen N.N'-Bis-salicyloyl-hydrazin (85% Ausbeute) und 230 Gew.-Teilen Paraffin. (Durch Lösen des Paraffins mit Methylenchlorid und Auskochen des Feststoffes mit Methanol wird N.N'-Bis-salicyloyl-hydrazin mit einem Schmelz- und Zersetzungsbereich von 305—310°C erhalten.)

Beispiel 4

10 Mol Hydrazinhydrat werden mit 20 Mol Arylcarbonsäurealkylester und 2,7 kg flüssigem Paraffin unter Schutzgas in der erfindungsgemäßen Apparatur erhitzt. Das bei einsetzender Reaktion entstehende Alkohol-Wasser-Gemisch wird kontinuierlich über eine Kolonne aus dem Reaktionsgemisch abdestilliert. Nach ca. 1—3 Stunden ist die Reaktionstemperatur auf über 100°C gestiegen und die Hälfte der theoretisch berechneten Menge des bei vollständiger Umsetzung freiwerdenden Alkohols und das gesamte, im Hydrazinhydrat enthaltene Wasser abdestilliert.

Nach Zugabe von 30 g Boroxid wird auf Temperaturen von 150 bis 200°C erhitzt. Der restliche entstehende Alkohol wird abdestilliert. Nach ca. 2—3 Stunden ist die Reaktion abgeschlossen, erkenntlich an der abdestillierten Menge Alkohol.

Die Apparatur wird nun evakuiert, das Bodenventil geöffnet und der Kristallbrei mit Hilfe des Dispergators und der Entgasungseinrichtung homogenisiert und von flüchtigen Bestandteilen befreit. Während dieses Prozesses wird die Temperatur der Paste auf 50—60°C erniedrigt. Danach wird mit Alkohol versetzt und die Mischung ca. 10 min bei 50—60°C kräftigst gerührt. Die gelb gefärbte Alkoholphase wird von der zähflüssigen Paste separiert und der Vorgang bis zur Entfärbung der Paste wiederholt.

Nach dem Abtrennen des Waschalkohols wird evakuiert und bei ca. 100°C getrocknet. Es werden nahezu theoretische Ausbeuten einer jeweils farblosen, zähflüssigen Paste erhalten. Durch Lösen des Paraffins mit Methylenchlorid und Auswaschen des zurückbleibenden Feststoffes mit Methanol werden die in der Tabelle aufgeführten Produkte der folgenden Formel in den angegebenen Ausbeuten erhalten.

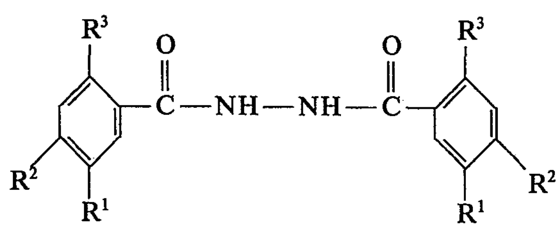

| $R^1$ | $R^2$ | $R^3$ | Ausbeute in % | Fp °C |
|---|---|---|---|---|
| H | H | H | 92 | 235 |
| Cl | H | OH | 87 | 345 |
| H | $OCH_3$ | OH | 91 | 310 |

## Patentansprüche

1. Verfahren zur Herstellung von N.N'-Bis-aroyl-hydrazinen durch katalytische Umsetzung von Arylcarbonsäurealkylestern mit Hydrazin oder Aroylhydrazinen in der Hitze, dadurch gekennzeichnet, daß die Reaktionspartner, nämlich Hydrazin bzw. Aroylhydrazin und Arylcarbonsäurealkylester, in nahezu stöchiometrischen Mengenverhältnissen in einem inerten, bei Reaktionsbedingungen flüssigen bis wachsartigen aliphatischen Kohlenwasserstoff geringer Flüchtigkeit zu einem pastenförmigen Produkt umgesetzt werden und dieses durch Dispergieren homogenisiert und unter Vakuum von den flüchtigen Reaktionskomponenten befreit wird.

2. Verfahren nach Anspruch 1, dadurch gekennzeichnet, daß als Reaktionspartner Hydrazin bzw. Salicylsäurehydrazid und Salicylsäurealkylester eingesetzt werden.

3. Verfahren nach Anspruch 1 und 2, dadurch gekennzeichnet, daß anschließend das pastenförmige Reaktionsprodukt durch Extraktion gereinigt wird.

4. Verfahren nach Anspruch 3, dadurch gekennzeichnet, daß mit einem aliphatischen Alkohol bis $C_5$ extrahiert wird.

5. Verfahren nach Anspruch 1 oder 2, dadurch gekennzeichnet, daß als inertes Medium Polyisobuten verwendet wird.

6. Verfahren nach Anspruch 1 oder 2, dadurch gekennzeichnet, daß als inertes Medium Paraffin verwendet wird.

7. Verfahren nach Anspruch 1 bis 6, dadurch gekennzeichnet, daß als Katalysator Bortrioxid verwendet wird.

8. Vorrichtung zur Durchführung des Verfahrens gemäß Anspruch 1 bis 7, dadurch gekennzeichnet, daß die Umsetzung von Hydrazin bzw. Aroylhydrazin und Arylcarbonsäurealkylester zu N.N'-Bis-aroylhydrazinen und deren Reinigung in einem inerten, bei Reaktionsbedingungen flüssigen bis wachsartigen Medium geringer Flüchtigkeit in einem Reaktor (1) erfolgt mit einer Schleuderscheibe (13), der verbunden ist mit Einrichtungen (2) zum Umpumpen, Abtrennen und Abfüllen der Reaktionskomponenten und Reaktionsprodukte und einem Dispergator (11) und gegebenenfalls einem dazwischengeschalteten Separator (15).

## Claims

1. Process for the production of N.N'-bis-aroyl hydrazines by thermal catalytic reaction of arylcarboxylic acid alkyl esters with hydrazine or aroylhydrazines, characterised in that the reaction partners, namely hydrazine or aroylhydrazine, as the case may be, and arylcarboxylic acid alkyl ester, in substantially stoichiometrical proportions, are converted into a paste-like product in an inert aliphatic hydrocarbon of negligible volatility which is in a liquid to wax-like state under the reaction conditions, and said product is homogenized by dispersion and freed from volatile reaction components under vacuum.

2. A process as claimed in Claim 1, characterised in that hydrazine or salicylic acid hydrazide and salicylic acid alkyl ester are used as reaction partners.

3. A process as claimed in Claim 1 and 2, characterised in that the paste-like reaction product is subsequently purified by extraction.

4. A process as claimed in Claim 3, characterised in that extraction is carried out with an aliphatic alcohol up to $C_5$.

5. A process as claimed in Claim 1 or 2, characterised in that polyisobutene is used as inert medium.

6. A process as claimed in Claim 1 or 2, characterised in that paraffin is used as inert medium.

7. A process as claimed in Claim 1 to 6, characterised in that boron trioxide is used as catalyst.

8. Apparatus for carrying out the process as claimed in Claim 1 to 7, characterised in that the reaction of hydrazine or aroylhydrazine and arylcarboxylic acid alkyl ester into N.N'-bis-aroyl-hydrazines and the purification thereof, is effected in an inert medium of negligible volatility which is in the liquid to wax-like state under the reaction conditions, in a reactor (1) by means of a centrifugal disc (13), said reactor being connected to devices (2) for pumping round, separating and drawing off the reaction components and reaction products, and a disperser (11), and, if necessary, a separator (15) inserte therebetween.

## Revendications

1. Procédé de préparation de N,N'-bis-aroyl-hydrazines, par réaction catalytique à chaud d'esters alcoyliques d'acides arylcarboxyliques sur l'hydrazine ou sur des aroylhydrazines, caractérisé en ce qu'il consiste à mettre à réagir les substances prenant part à la réaction, à savoir l'hydrazine ou une aroylhydrazine et un ester alcoylique d'acide arylcarboxylique, en des rapports quantitatifs presque stoechiométriques, dans un hydrocarbure aliphatique inerte de faible volatilité, qui est liquide à cireux dans les conditions réactionnelles, pour obtenir un produit pâteux, et à homogénéiser celui-ci par

dispersion, et à le débarrasser sous vide des constituants réactionnels volatils.

2. Procédé suivant la revendication 1, caractérisé en ce qu'il consiste à mettre en œuvre, comme substances prenant part à la réaction, de l'hydrazine ou de l'hydrazide d'acide salicylique et un salicylate d'alcoyle.

3. Procédé suivant la revendication 1 ou 2, caractérisé en ce qu'il consiste ensuite à purifier le produit de réaction pâteux par extraction.

4. Procédé suivant la revendication 3, caractérisé en ce qu'il consiste à effectuer l'extraction par un alcool aliphatique ayant jusqu'à 5 atomes de carbone.

5. Procédé suivant la revendication 1 ou 2, caractérisé en ce qu'il consiste à utiliser comme milieu inerte du polyisobutène.

6. Procédé suivant la revendication 1 ou 2, caractérisé en ce qu'il consiste à utiliser comme milieu inerte une paraffine.

7. Procédé suivant l'une des revendications 1 à 6, caractérisé en ce qu'il consiste à utiliser comme catalyseur du trioxyde de bore.

8. Appareil pour exécuter le procédé suivant les revendications 1 à 7, caractérisé en ce que la réaction de l'hydrazine, ou d'une aroylhydrazine sur un ester alcoylique d'acide arylcarboxylique pour donner des N,N'-bis-aroylhydrazines et leur purification dans un milieu inerte de faible volatilité, qui est liquide à cireux dans les conditions réactionnelles, s'effectue dans un réacteur (1) comprenant un disque de centrifugation (13), qui communique avec des dispositifs (2) pour faire recirculer par pompage, séparer et décharger les constituants réactionnels et les produits de la réaction, et avec un appareil à disperser (11) et, le cas échéant, avec un séparateur (15) montés entre eux.

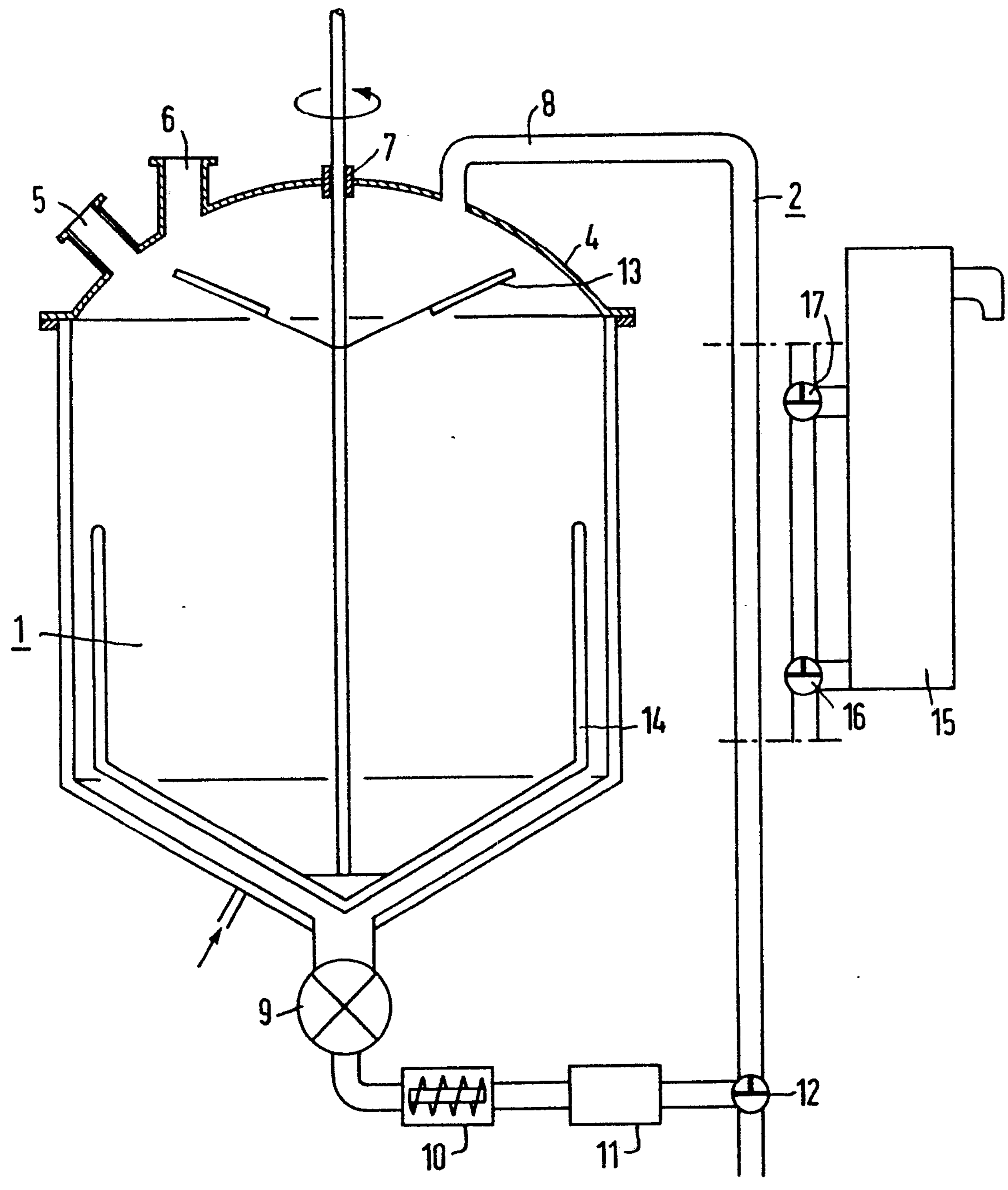

8
6
7
5
2
4
13
17
1
14
16
15
9
12
10
11